# EUROPEAN PATENT APPLICATION

(11) **EP 2 014 296 A1**
(43) Date of publication of application: **14.01.2009**
(21) Application number: 07013480.4
(22) Date of filing: 10.07.2007
(51) Int. Cl.: A61K 38/00, A61P 9/10, A61P 7/02

(54) **Novel strategies for increasing the reperfusion in obstructed blood vessel**

(71) Applicant: PAION Deutschland GmbH, 52062 Aachen (DE)
(72) Inventor: Petersen, Karl-Uwe, 52072, Achen (DE); Brohmann, Henning, 40589, Düsseldorf (DE); Lawrence, Daniel A., Ann Arbor MI 48109.0644 (US)
(74) Representative: Von Renesse, Dorothea

(57) **Abstract**

Use of thrombomodulin for the manufacture of a medicament for stimulating the reperfusion of an ischemic tissue or organ within a patient in the need thereof, in particular a stroke patient.

## Description

Cellular function critically hinges on a sufficient supply of oxygen by the bloodstream. Thus, obstruction of blood supply may, depending on its extent, result in a damage or dysfunction of the downstream tissues. This inadequate flow of blood or shortage of blood supply to an organ or tissue is referred to as ischemia. One of the most prominent reasons underlying an ischemic event is a thrombus formation in a blood vessel.

Insufficient provision with oxygen means that tissues become hypoxic, or, if no oxygen is supplied at all, anoxic, a condition that, if prevailing for too long will cause necrosis (i.e. cell death).

Ischemia is *inter alia* a feature of certain heart diseases, transient ischemic attacks, cerebrovascular accidents, rupture of arteriovenous malformations and/or peripheral artery occlusive diseases. Tissues especially sensitive to inadequate blood supply include the heart, the kidneys and the brain. Ischemia in brain tissue, for example due to a stroke or head injury, causes the so-called ischemic cascade to be unleashed, which stands for a series of consecutive, parallel and interacting biochemical events involving proteolytic enzymes, reactive oxygen species, and other active compounds. The final outcome of this sequence is damage and destruction of brain tissue.

Stroke, a synonym for ischemic events in brain tissue, is the third leading cause of death, after cardiovascular disease and cancer. Each year, stroke is diagnosed in 750.000 patients and contributes to nearly 168.000 deaths in the United States only. Stroke has a high personnel and social impact because of the severe disability that the disease causes.

Recanalization of occluded arteries to restore blood supply is the most effective therapy for stroke treatment in humans to date. The success rate of such treatment is reduced by the possibilities of intracerebral hemorrhage and reocclusion, Hemorrhage is part of the damage caused by the ischemic cascade, but is also a risk associated with thrombolytic agents. Reocclusion may occur at the same site or by fragments of the original clot that have been carried downstream. In a recent study (Rubiera et al., Predictors of early arterial reocclusion after tissue plasminogen activator-induced re-canalization in acute ischemic stroke; Stroke 2005; 36:1452-1456) reocclusion occurred in 84 of 142 (61%) consecutive stroke patients (53 partial, 31 complete). Of these, 21 (25%) patients worsened after an initial improvement.

In general, a known method for prevention of obstruction or reobstruction (reocclusion) of vessels is to treat a thrombotic patient with an anticoagulant or antiplatelet drug. Anticoagulants are substances, which reduce the ability of blood to form blood clots. Various anticoagulants are known to the skilled person, for example heparin or warfarin.

Although the anticoagulant or anti-thrombotic effects of various substances are known, currently there is not any approved anticoagulant therapy for treating acute stroke patients. Of the numerous antiplatelet drugs, only low-dose aspirin is recommended. However, according to the guidelines of the American Heart Association (AHA) and the American Stroke Association (ASA), this recommendation is limited to the first 24-48 hours after acute ischemic stroke (see Guidelines, May 2007. page 1681, left column).

Regarding anticoagulation therapy the guidelines explicitly state that
- urgent anticoagulation with the goal of preventing early recurrent stroke, halting neurological worsening, or improving outcomes after acute stroke is not recommend for treatment of patients with acute ischemic stroke,
- urgent anticoagulation is not recommend for patients with moderate and severe strokes because of an increased risk of serious intracranial hemorrhagic complications
- the initiation of anticoagulant therapy within 24 hours of treatment with intravenously administered tPA is not recommended (Guidelines, page 1680. left column).

The reluctance of scientists and regulatory authorities with respect to anticoagulant stroke therapy is merely due to the fact, that anticoagulants have been shown to increase the risk for hemorrhagic transformation in stroke patients, potentially obliterating any therapeutic benefit. The anticoagulant therapies approved so far are limited to the fields of cardiac infarction and pulmonary embolism.

However, according to the invention it has now been found that thrombomodulin can be used to improve or even effect reperfusion of obstructed blood vessels, in particular obstructed blood vessels of stroke patients. This improvement is not necessarily linked to an increase in the risk of hemorrhagic transformation.

Thrombomodulin is a membrane protein that acts as a thrombin receptor on the endothelial cells lining the blood vessels. Thrombin is a central enzyme in the coagulation cascade, which converts fibrinogen to fibrin, the matrix clots are made of. Initially, a local injury leads to the generation of small amounts of thrombin from its inactive precursor, prothrombin. Thrombin, in turn, activates platelets and, second, certain coagulation factors including factors V and VIII. The latter action gives rise to the so-called thrombin burst, a massive activation of additional thrombin molecules, which finally results in the formation of a stable clot.

When bound to thrombomodulin, however, the activity of thrombin is changed in direction: A major feature of the thrombin-thrombomodulin complex is its ability to activate protein C, which then downregulates the coagulation cascade by proteolytically inartivating the essential cofactors Factor Va and Factor VIIIa (Esmon et al., Ann. N. Y. Acad. Sci. (1991), 614:30-43), thus affording anticoagulant activity. The thrombin-thrombomodulin complex is also able to activate the thrombin-activatable fibrinolysis inhibitor (TAFI), which then antagonizes fibrinolysis.

Although earlier studies were negative, more recent studies have indicated that thrombomodulin is not only present in brain endothelial cells (Boffa, et al., Nouv. Rev. Fr. Hematol. (1991), 33:423-9; Wong, et al., Brain Res. (1991), 556:1-5; Wang, et al., Arte-rioscler. Thromb. Vasc. Biol. (1997), 17: 3139-46: Tran, et al., Stroke (1996), 27:2304-10; discussion 2310-1) but is also expressed on the surface of astrocytes, where it functions as in the vasculature, namely activating protein C after forming a complex with thrombin (Pindon, et al., Glia (1997), 19:259-68). Thrombomodulin is also upregulated in reactive astrocytes in the CNS, in response to mechanical injury (Pindon, et al., J. Neurosci. (2000), 20:2543-50). A pertinent report suggests that recombinant thrombomodulin is capable of blocking thrombin's activation of another receptor, the protease-activated receptor 1 (PAR-1) in cultured neuronal cells (Sarker, et al. Thromb, Haemost. (1999), 82: 1071-77).

Activated protein C has also been strongly implicated in the regulation of inflammatory responses involving various cytokines or activated leukocytes (Esmon et al., Thromb. Haemost. (1991), 66:160-165). Consistent with this hypothesis, studies have shown that activated protein C, by inhibiting the production of tumor necrosis factor (TNF-alpha) prevents pulmonary vascular injury in rats administered endotoxin TNF-alpha is a potent activator of neutrophils (Murakami et al., Blood (1996), 87:642-647; Murakami et al., Am. J Physiol. (1996), 272:L197-2). This is consistent with the ability of recombinant human soluble thrombomodulin to prevent endotoxin-induced pulmonary vascular injury by inhibiting the activation of neutrophils, in an action mediated by protein C activation (Uchiba et al., Am. J. Physiol, (1996), 271 :L470-5; Uchiba et al., Am. J Physiol. (1997), 273:L889-94).

The effect of thrombomodulin according to one embodiment of the invention regarding the improvement of reperfusion was surprisingly found in an animal model with a stable occlusion of the middle cerebral artery (MCA). Mice with an occluded MCA were treated with a soluble thrombomodulin and surprisingly exhibited a substantial reperfusion as measured by an increase cerebral blood flow (CBF). This finding was unexpected, since the anticoagulant effects known for thrombomodulin from animal studies were merely suggestive of an ability to prevent blood clotting, rather than to restore blood flow.

In the context of the present invention the term reperfusion refers to any improvement of the blood flow compared to the state of occlusion, which can either be a partial or a total reperfusion. Thus the term reperfusion is not limited to effects, which result in the complete restoration of the original blood flow before the ischemic event. According to the invention, the reperfusing effect of thrombomodulin is assumed to include effects which antagonize the thrombus formation and/or support the spontaneous lysis of blood clots.

In another embodiment of the invention, the thrombomodulin is used for preventing or at least lowering the risk of an occlusion of blood vessels by thromboembolic events, in particular by secondary thromboembolic events. These secondary thromboembolic events can be cerebral blood clots, deriving from the primary ischemic event. Thus, according to this embodiment of the invention, thrombomodulin can be used to treat stroke patients suffering from the risk of secondary cerebral vascular occlusions (reocclusions).

It is known to the skilled person, that currently no anticoagulant therapy for preventing a reocclusion with anticoagulants is approved, since there is an incalculable risk of intracerebral hemorrhage, which is even accentuated after the administration of a thrombolytics. The novel teaching according to one embodiment of the invention thus is that thrombomodulin can be administered to an ischemic patients (in particular stroke patients) with less or no substantial risk of deleterious effects, in particular bleeding, compared to patients untreated with thrombomodulin. Hence, even if the patient had suffered a stroke before and/or had received thrombolytic therapy, he can be subject to anticoagulation therapy with soluble thrombomodulin according to one embodiment of the invention. The beneficial effect of thrombomodulin regardless of a possible prior thrombotic or ischemic event or a thrombolytic therapy, was unexpected to the skilled person, since studies reported an association between an increased level of soluble thrombomodulin and an increased risk of death in patients with stroke (Olivot JM, et al; Soluble Thrombomodulin and Brain Infarction. in: Stroke 2004, 35:1946-1951).

In yet another aspect of the invention, the thrombomodulin is administered to the patient within the acute or early phase of the ischemic event. In a particular embodiment, the administration is applied in the time window of the first 24 hours after the administration of a thrombolytic substance, for example tPA or any other plasminogen activating factor.

In a further embodiment of the invention, the thrombomodulin as administered within three hours after the onset of the ischemic event.

According to yet another aspect of the invention the thrombomodulin is administered to the patients in a dosage, which does not substantially increase the blood coagulation parameters, as measured for example by an APTT or PTT assay known to the skilled person in the art. The APTT (Activated Partial Thromboplastin Time) or PTT (Partial Thromboplastin Time) is an assay which is indicative of the efficacy of the intrinsic and the common coagulation pathway, respectively. Apart from detecting abnormalities in blood clotting, it is also used to monitor the treatment effects of anticoagulants.

According to another embodiment of the invention, a dosage of thrombomodulin is administered to the patient, which is not associated with a prolonged coagulation time. The APTT values of healthy humans lie normally between around 25 sec. and 39 sec. Values outside this range are generally considered as abnormal. According to this aspect of the invention, the applied dosage does not substantially prolong the APTT, but effectively prevents vessel occlusion as measured e.g. in the assay of photothrombotic occlusion of the middle cerebral artery in the mouse.

The preferred dosage of thrombomodulin according to the invention is approximately 0.01 to 5 mg/kg body weight, preferably 0.03 to 3 mg/kg body weight, most preferred 0.03 to 1.5 mg/kg body weight or 0.05 to 1.0 mg/kg body weight. Thus according to the invention, a possible dosage unit form, such as a vial or ampoule, is suggested containing around 0.8 to 400 mg, preferred 2.4 to 240 mg, most preferred 0.8 to 1200 mg or 4 to 80 mg (assuming a patient of around 65 kg body weight).

The methods according to the invention can be performed with any soluble thrombomodulin. Thrombomodulin in the context of the present invention is defined as any protein, including thrombomodulin modifications, analogues, variants, fragments and derivatives thereof, substantially showing the biological activity of native thrombomodulin regarding the capacity to form a functional complex with thrombin (all commonly referred to herein as thrombomodulin). According to the invention a "functional complex' with thrombin is any complex, which activates the protein C / protein S pathway.

Various forms of soluble thrombomodulin are known to the skilled person, e.g. the so called ART-123 developed by Asahi Corporation (Tokyo, Japan) or the recombinant soluble human thrombomodulin Solulin, currently under development by PAION Deutschland GmbH, Aachen (Germany). The recombinant soluble thrombomodulin, i.e. a soluble thrombomodulin without a modification of the amino acid sequence, is subject of the Asahi patent EP0 312 598

Solulin is a soluble, as well as protease and oxidation-resistant analogue of human thrombomodulin and thus exhibits a long life *in vivo.* Solulin's main feature lies in its broad mechanism of action since it not exclusively inhibits thrombin. It also activates the natural protein C / protein S pathway, and therefore stops further generation of thrombin. Furthermore, Solulin exerts a neuroprotective effect (see e.g. EP 1 365 788). since binding of Solulin to thrombin prevents the latter from activating its Protease Activatable Receptors (PARs). Inhibition of PAR receptors in turn blocks apoptotic cell death.

Solulin is *inter alia* subject of the European patent 0 641 215 B1, EP 0 544 826 B1 as well as EP 0 527 821 B1.

According to one embodiment of the invention, Solulin is administered to the patient. Solulin contains modifications compared to the sequence of mature recombinant soluble thrombomodulin (SEQ. ID NO. 1) at the following positions: Removal of amino acids 1-3, M833L, R456G, H457Q, S474A and termination at P490. This numbering system is in accordance with the native thrombomodulin of SEQ. ID NO. 1. The sequence of Solulin as the preferred embodiment of the invention is shown in SEQ. ID NO. 2.

However, notably, according to the invention also thrombomodulin analogues can be utilized, which comprise only one or more of the above mentioned properties, or of the properties outlined in the above mentioned European patent documents EP 0 544 826 B1, EP 0 641 215 B1 and EP 0 527 821 B1.

Particularly preferred thrombomodulin variants applicable according to the invention are those that have one or more of the following characteristics:
(i) they are oxidation resistant,
(ii) they exhibit protease resistance,
(iii) they have homogeneous N- or C-termini,
(iv) they have been post-translationally modified, e.g., by glycosylation of at least some of the glycosylation sites of native thrombomodulin (SEQ ID NO: 1),
(v) they have linear double-reciprocal thrombin binding properties,
(vi) they are soluble in aqueous solution in relatively low amounts of detergents and typically lack a transmembrane sequence,
(vii) they are lacking a glycosaminoglycan chain.

The manufacture of these analogues used in this invention is disclosed in the above mentioned European patent documents.

Most preferred is a molecule comprising all of these modifications, e.g. as shown by Solulin.

In a further embodiment of the invention, the thrombomodulin analogue known from the WO 01/98352 A2 can be used. In another embodiment of the invention rabbit derived thrombomodulin can be used. The 6EGF fragment of Solulin is an example of a thrombomodulin fragment with essentially the same biological activity regarding the formation of a complex with thrombin with the ability to activate human protein C. This fragment essentially consists of the six epithermal growth factors domain of native thrombomodulin.

### I. EXAMPLE

### Photothrombotic Model of Ischemic Stroke: Thrombomodulin Added Prior to Arterial Occlusion

Using a model of photothrombotic occlusion of the middle cerebral artery the ability of soluble thrombomodulin (Solulin) to interfere with thrombosis was tested. The drug was added prior to occlusion of the middle cerebral artery.

### 1. Test system

The study was conducted in male mice, C57BL/6 strain. The average body weight of the animals at the time of allocation was 26 grams. The age of the animals was approximately 10 weeks.

### 2. Experimental procedures Stroke model

The mice were anesthetized with 90mg/kg intraperitoneal chloral hydrate Morton Grove Pharmaceutical Morton Grove, IL) and then placed securely under a dissecting microscope (Nikon SMZ-2T, Mager Scientific, Inc.). After exposing the left middle cerebral artery (MCA), a laser Doppler flow probe (Type N (18 gauge), Transonic Systems) was attached to the surface of the exposed skull over the cerebral cortex located 1.5 mm dorsal median from the bifurcation of MCA. The probe was connected to a flowmeter (Transonic model BLF21) and records were made using a continuous data acquisition program (Windaq, DATAQ Instruments). A 1.5 mW green light laser (540 nm, Melles Griot) was directed at the MCA from a distance of 6 cm and Rose Bengal (Fisher Scientific) diluted to 10 mg/mL in PBS was then injected into the tail vein with the final dose of 50 mg/kg. The laser was then continued for 10 minutes after occlusion. The tissue perfusion rate of the cerebral cortex was monitored continuously with the laser doppler flow meter and recorded for approximately 2-3 hours. Stable occlusion was achieved if the tissue perfusion rate was reduced by 70 % or more of its original value.

### Study treatment and observations

30 min before induction of thrombus formation by laser irradiation, rats received bolus injections of Solulin (1 or 3 mg/kg) or vehicle in equal volumes (100µl/kg). The time to thrombus formation was assessed by Laser Doppler measurement. The laser measurements were continued for at least 150 min after injection of Solulin or vehicle. The experiments were terminated approximately 2 hours after Solulin or vehicle administration by sacrificing the surviving animals

### Data analysis

Tissue perfusion units (TPU) were recorded using DATAQ Instruments Win DAQ Serial Acquisition.

### Occlusion time

Time needed for occlusion was calculated as the time between injection of Bengal red and stable occlusion. The latter was assumed when a 70% or higher reduction of blood flow had prevailed for 5 min. Times were read directly off the tracing and converted to number format.

### Determination of blood flow

Blood flow data was compressed (- 20 points per second) and saved as a CSV file. Raw data was graphed and irregular points were deleted. The flow data was normalized to a percentage using the average TPU value from 10 minutes before Rose Bengal injection to the injection. Area under the curve was calculated from the normalized flow from the Rose Bengal injection to 120 minutes using GraphPad Prism. In cases in which blood flow data became unavailable for technical reasons or death of the animal, a value of zero was assumed from that time point on.

### Statistics

A two tailed tTest was used to determine significance. For each test measure, probability values of p<0.05 were considered statistically significant.

### 3. Results

### Safety

A total of 35 mice were used. Bengal red, which by itself poses substantial stress on the animals, caused fatalities in 3 animals, which died before middle cerebral artery occlusion was obtained (2/14 in controls, 0/10 in the low and 1/11 in the high Solulin dose group). These mice were not considered for evaluation.

Solulin was generally well tolerated at either dose level. Fatalities after arterial occlusion in the control and the low and high dose Solulin groups were 0/12, 0/10, and 1/10 animals, respectively

### Time course of blood flow in the middle cerebral artery

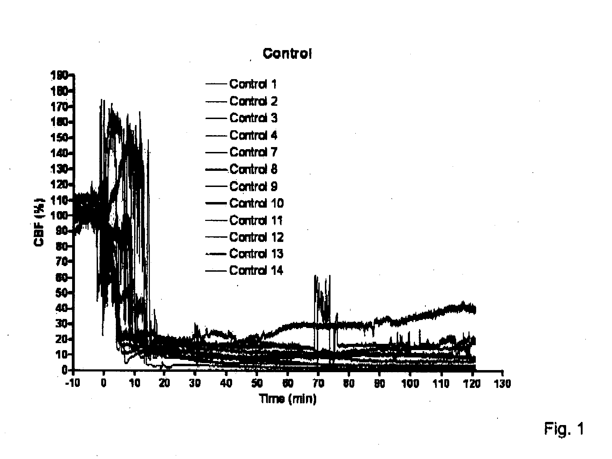

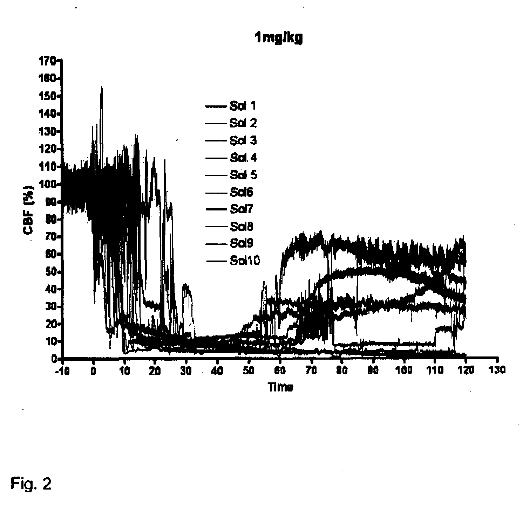

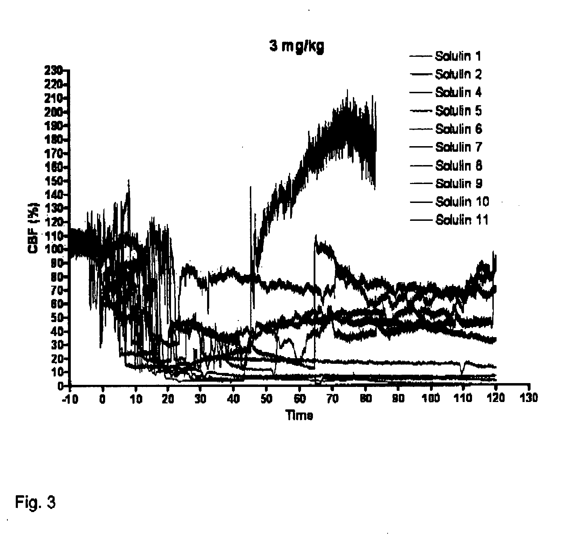

Time courses of blood flow in the middle cerebral artery are shown in Figures 1-3. Traces are shown after normalization to a percentage based on the TPU value averaged from 10 minutes before Rose Bengal injection to the injection. The time to occlusion as indicated by the drop of blood flow was prolonged in Solulin-treated animals compared to control mice. More striking, the occlusion remained stable in control experiments (except for one technical perturbation, see legend to Figure 1) while it was transient in most Solulin-treated animals (4/10 and 6/10 animals at 1 and 3 mg/kg Solulin, respectively).

### Time to occlusion

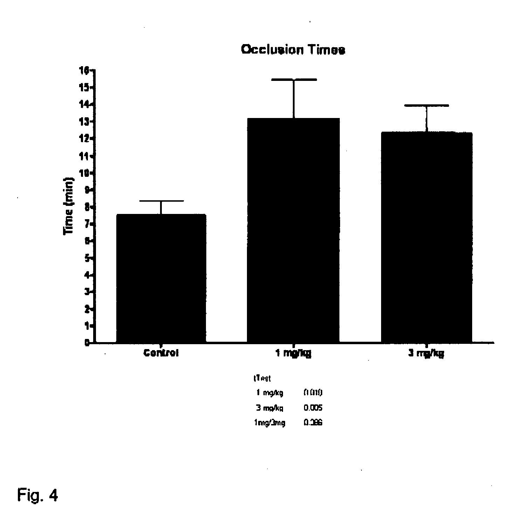

Figure 4 summarizes the time needed for occlusion. Solulin at either dose significantly prolonged the time to occlusion compared to controls. (The P values of 1 mg/kg and 3 mg/kg versus control are 0.01 and 0.005, respectively. Shown are mean ± SEM of 10-12 animals per group).

### Blood flow (reperfusion)

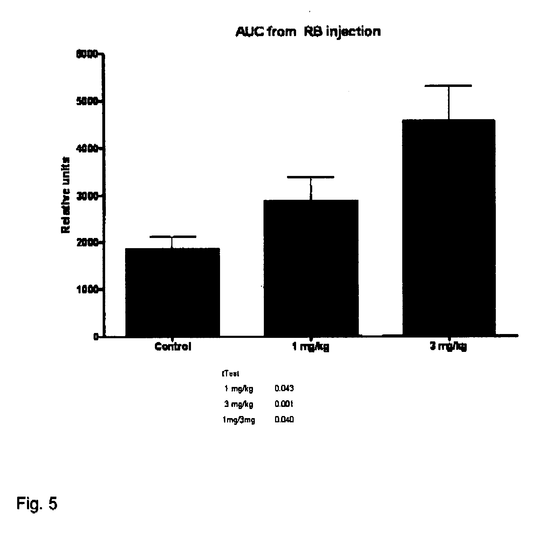

Blood flow (reperfusion) was quantitated by determination of AUC of the blood flow vs. time chart over the time from the Rose Bengal injection to 120 minutes (Figure 5). AUC obtained with the two Solulin concentrations was significantly larger than in vehicle-treated mice; also, the AUC at 3 mg/kg significantly exceeded that at 1 mg/kg. In the Solulin high dose group, one of the animals developed an exceedingly high level of reperfusion and deceased thereafter. However, exclusion of this animal from assessment had no impact on the data and the conclusions, as the AUC with and without that animal amounted to 45623and 38474 relative units, respectively, which was still significantly different from the values in the control group and at the dose of 1 mg/kg.

### Legend

**Fig. 1** **Tissue perfusion downstream of the middle cerebral artery in controls**
   Thirty min after injection of vehicle, Rose Bengal was injected into the tail vein (time 0 in the diagram) to induce thrombus formation and subsequent occlusion at the site of laser irradiation. Animals 5 and 6 died shortly after the Rose Bengal injection and were not included in this data analysis. The temporary increase in the tracing of one animal may be related to insufficient anaesthesia resulting in movement of the animal relative to the laser flow probe. However, this temporary increase, regardless of its cause, was still included in the analysis.
**Fig. 2** **Tissue perfusion downstream of the mouse middle cerebral artery after injection of Solulin at a dose of 1 mg/kg**
   Thirty min after injection of Solulin, Rose Bengal was injected into the tail vein (time 0 in the diagram) to induce thrombus formation and subsequent occlusion at the site of laser irradiation.
**Fig. 3** **Tissue perfusion downstream of the mouse middle cerebral artery after injection of 3 mg/kg Solulin**
   Thirty min after injection of Solulin, Rose Bengal was injected into the tail vein (time 0 in the diagram) to induce thrombus formation and subsequent occlusion at the site of laser irradiation. Animal 3 died shortly after the Rose Bengal injection and was not included in the analysis. Animal 6, which displayed an increase in blood flow beyond 100% after initial occlusion, died 85 minutes after injection of Rose Bengal.
**Fig. 4****. Time needed for occlusion in the presence of vehicle or Solulin**
   The difference between control and Solulin was significant at either concentration designated by the single asterisk. The P values at 1 mg/kg and 3 mg/kg versus control are 0.010 and 0.005, respectively. Shown are mean ± SEM of 10-12 animals per group.
**Fig. 5** **Area under the curve of the blood-flow time charts from the Rose Bengal injection to 120 minutes afterwards**
   The difference between Solulin and controls was significant at either Solulin concentration and so was the difference between the 1 and 3 mg/kg Solulin doses. The P values of 1 mg/kg and 3 mg/kg versus control were 0.043 and 0.001, respectively. The P value of 1 mg/kg versus 3 mg/kg was 0.040. Data are presented in relative units representing the area under the curve of the blood-flow time chart per 120 min observation interval. Shown are mean values ± SEM of 9-12 animals per group.

### II. EXAMPLE

### Photothrombotic Model of Ischemic Stroke: Thrombomodulin Added after Arterial Occlusion

Using a model of photothrombotic occlusion of the middle cerebral artery the ability of soluble thrombomodulin (Solulin) to interfere with thrombosis was tested. The drug was added after occlusion of the middle cerebral artery.

### 1. Test system

The study was conducted in mice, C57BU6 strain. The age of the animals at the start of testing was approximately 12 weeks.

### 2. Experimental procedures

### Stroke model

The mice were anesthetized with 90 mg/kg intraperitoneal chloral hydrate (Morton Grove Pharmaceutical Morton Grove, IL) and then placed securely under a dissecting microscope (Nikon SMZ-2T, Mager Scientific, Inc.). After exposing the left middle cerebral artery (MCA), a laser Doppler flow probe (Type N (18 gauge), Transonic Systems) was attached to the surface of the exposed skull over the cerebral cortex located 1.5 mm dorsal median from the bifurcation of MCA. The probe was connected to a flowmeter (Transonic model BLF21) and recorded with a continuous data acquisition program (Windaq, DATAQ Instruments), A 1.5 mW green light laser (540 nm, Melles Griot) was directed at the MCA from a distance of 6 cm and Rose Bengal (Fisher Scientific) diluted to 10 mg/mL in PBS was then injected into the tail vein with the final dose of 50 mg/kg. The laser was then continued for 10 minutes after occlusion. The tissue perfusion rate of the cerebral cortex was monitored continuously with the laser doppler flow meter and recorded for approximately 2-3 hours. Stable occlusion was achieved if the tissue perfusion rate was reduced by 70% or more of its original value. After obtaining stable occlusion of the MCA for 30 min, treatment with the test substances was initiated and the cerebral blood flow (CBF) was continuously measured for up to 2 hours. A final CBF assessment for reocclusion was performed 72 h post stroke induction before animals were sacrificed.

### Administration of the test and control substances

A bolus injection of Solulin (100µl) was administered via the tail vein 30 minutes after a stable MCA occlusion has been verified (ischemic stroke). After stroke, further Solulin injections were performed at 24 hour intervals to a total of three. Animals were euthanized 72 hours post-stroke.

Successful lysis of clot was verified by a laser doppler flow probe set in the infarct region. Blood flow was determined in control and Solulin animals.

### Experimental groups and dose levels

The following table summarizes experimental groups and dosages:

| **Treatment** | **Animal Number** | **Dosage** |
|---|---|---|
| Control (Vehicle/Vehicle) | 15 | - |
| Solulin/Vehicle | 15 | 1 mg/kg |

The following chart the application schedules of the Solulin and control groups described above:

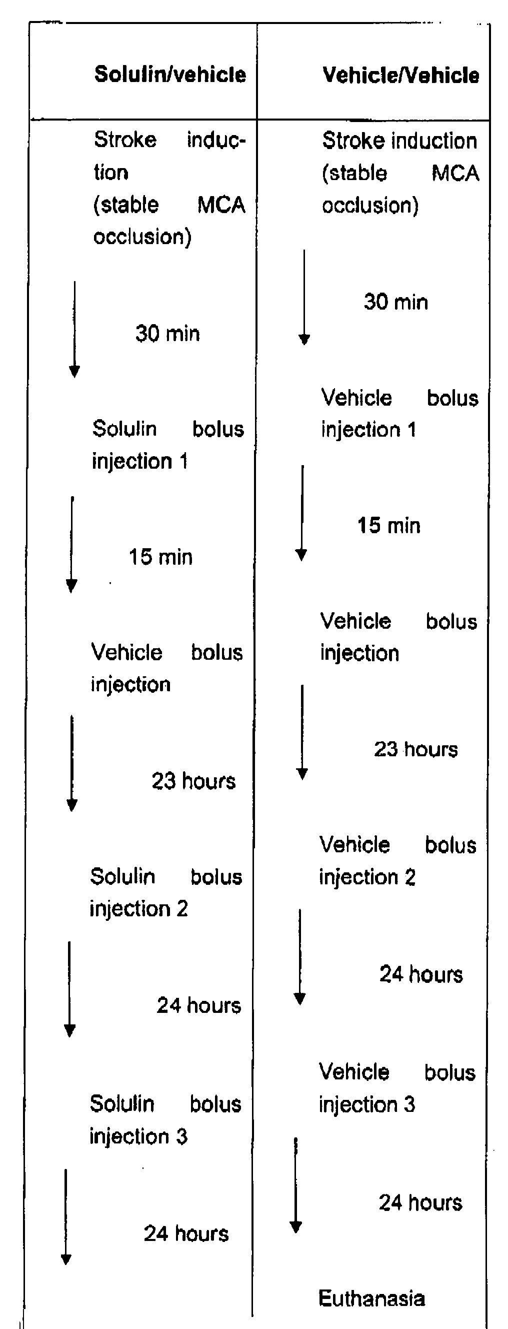

### Statistical analysis of the results

The statistical procedures used in the evaluation of data were as follows:
All data were analyzed by ANOVA (analysis of variances of the mean) between the treatment groups. p<0.05 was considered statistical significant.

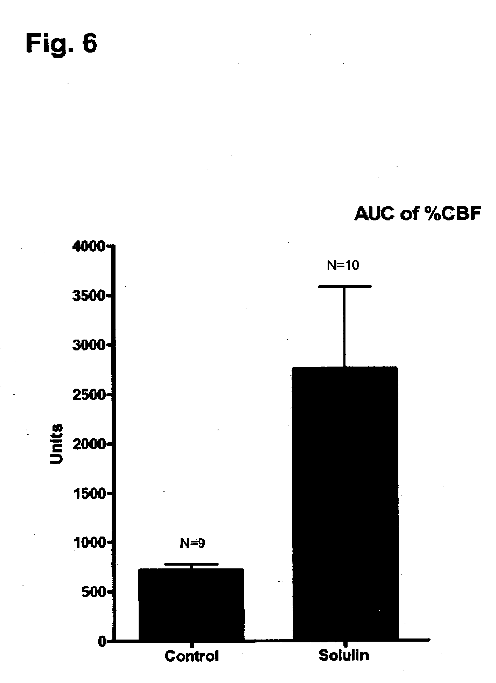

**Fig. 6** shows the AUC of Cerebral Blood Flow (CBF; % of 10 min reference period before Rose Bengal injection), followed for 2 hours. (Time of stable occlusion = 0; Blood flow followed for 2 hours: Solulin at 1 mg/kg).

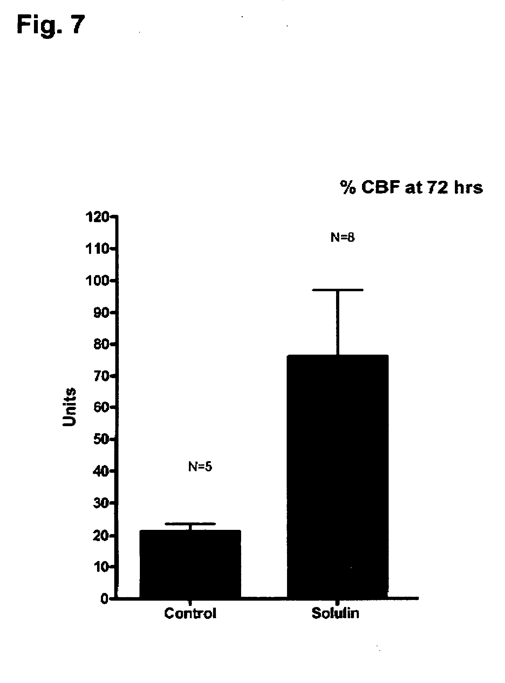

**Fig. 7** shows the CBF at 72 hours post-stroke. (Solulin a 1 mg/kg; Additional doses on day 2 and day 3).

### III. EXAMPLE

### Inhibition of venous thrombosis in a stasis model in rats

The acute anti-thrombotic efficacy of Solulin doses was studied in a rat model of thromboplastin-induced venous stasis, together with two comparators: activated protein C (APC), using the commercially available drug, Xigris^{®} (Drotrecogin alfa activated), and the low molecular weight heparin, Enoxaparin.

### 1. Test System and Experimental Procedures

The vena cava was prepared in anaesthetized male Sprague-Dawley rats and two loose ligatures were made on the vena cava between the left renal and the iliac vein. Solulin, Enoxaparin or vehicle (0.9% NaCl) were i.v. administered 30 min before i.v. injection of 250 µg/kg thromboplastin. APC was i.v. applied 5 min before thromboplastin. Injected volumes were 5 ml/kg in each case.

Thirty seconds after the end of the thromboplastin injection, stasis was established by tightening the sutures. After a period of 30 min, the formed thrombus was removed from the segment, scored on a scale of 1-4 (according to the Wessler technique meaning 0 = fluid blood, 1 = one or several small clots, 2 = non-occlusive thrombus filling 50 % of the vascular segment, 3 = non-occlusive thrombus filling 75 % of the vascular segment, 4 = occlusive segment), blotted on filter paper and weighed immediately (wet weight) and after drying at 37°C for 24 hours (dry weight). A decrease of 30 % or more in average thrombus weight or Wessler score relative to the vehicle-treated group was considered indicative of significant anti-thrombotic activity.

### 2. Results

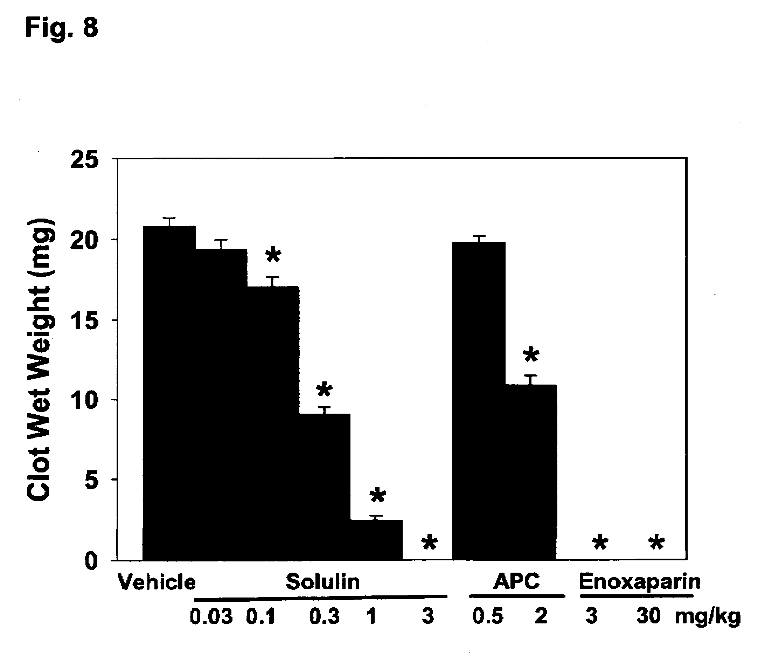

As evident from tab. 1 and Fig. 8 (Antagonism of Clot Formation (Ligated Rat Vena Cava in vivo, Thromboplastin-induced)), Solulin dose-dependently reduced thrombus sizes and weights, abrogating any thrombus formation at the 3 mg/kg high dose. Effects were significant starting from the dose of 0.1 mg/kg. By contrast, APC showed significant anti-thrombotic activity (~50 % reduction in thrombus weights and Wessler score) only at the higher dose tested (2 mg/kg), an effect comparable in magnitude to that of Solulin at 0.3 mg/kg. As summarized in the safety disussion, only APC was associated with sizable prolongation of APTT and bleeding time at this efficacy level. Enoxaparin suppressed thrombus formation at both dosages tested. Also these effects were associated with substantial prolongation of APTT and bleeding time.

**Table 1:Thrombus weights and Wessler score after Solulin, APC, Enoxaparin, or vehicle administration in a venous stasis model in rats.**

| **Substance** | | **Thrombus weight** | | | | **Wessler score** | |
|---|---|---|---|---|---|---|---|
| | **Dose [mg/kg]** | **wet weight [mg]** | **% reduction** | **dry weight [mg]** | **% reduction** | **Mean value** | **% reduction** |
| Vehicle | 5 ml/kg | 20.82 ±0.53 | - | 6.80 ± 0.21 | - | 4.0 ± 0.0 | - |
| Solulin | 0.03 | 19.39 ± 0.57 | 7 | 6.46 + 0.17 | 5 | 3.8 ± 0.1 | 5 |
| | 0.1 | 16.99 ± 0.64* | 18 | 5.66 ± 0.21* | 17 | 3.4 ± 0.2* | 15 |
| | 0.3 | 9.05 ± 0.46* | 57 | 3.10 ± 0.17* | 54 | 1.8 ± 0.1* | 55 |
| | 1 | 2.44 ± 0.30* | 88 | 0.83 ± 0.11* | 88 | 1.0 ± 0.0* | 75 |
| | 3 | 0* | 100 | 0* | 100 | 0* | 100 |
| APC | 0.5 | 19.74 ± 0.44 | 5 | 6.56 ± 0.20 | 4 | 3.8 ± 0.1 | 5 |
| | 2 | 10.90+ 0.59* | 48 | 3.62 ± 0.21* | 47 | 2.2 ± 0.1* | 45 |
| Enoxaparin | 3 | 0* | 100 | 0* | 100 | 0* | 100 |
| | 30 | 0* | 100 | 0* | 100 | 0* | 100 |

| | | | | | | | |
|---|---|---|---|---|---|---|---|
| * p < 0.05 by one-way ANOVA followed by Dunnett's test; % reduction compares the value to the vehicle group; data represent mean values ± SEM of 10 animals per group. | | | | | | | |

### IV. EXAMPLE

### Effect on tail transaction bleeding time in rats

The effect of Solulin compared to activated protein C (APC; using the commercially available drug, Xigris^{®}, Drotrecogin alfa activated) and Enoxaparin on tail transection bleeding time was analyzed in rats.

### 1. Test System and Experimental Procedures

Doses and application regimen were the same as in the efficacy study (rat stasis model of venous thrombosis, see example 3).

Solulin, Enoxaparin or vehicle were i.v. injected into anaesthetized male and female Sprague-Dawley rats 30 min before standardized transection of the tip (3 mm) of the tail. APC was i.v. administered 5 min prior to tail tip transection. All rats remained anesthetized during the observation period. Subaqueous bleeding time was determined in pre-warmed saline at 37°C by the length of time between vertical immersion of the tails into the test tube and bleeding cessation for at least 15 sec. Any incidences of re-bleeding were noted for up to 20 min thereafter. Prolongation of bleeding time by 50 % or more relative to the vehicle control group of animals was considered significant.

### 2. Results

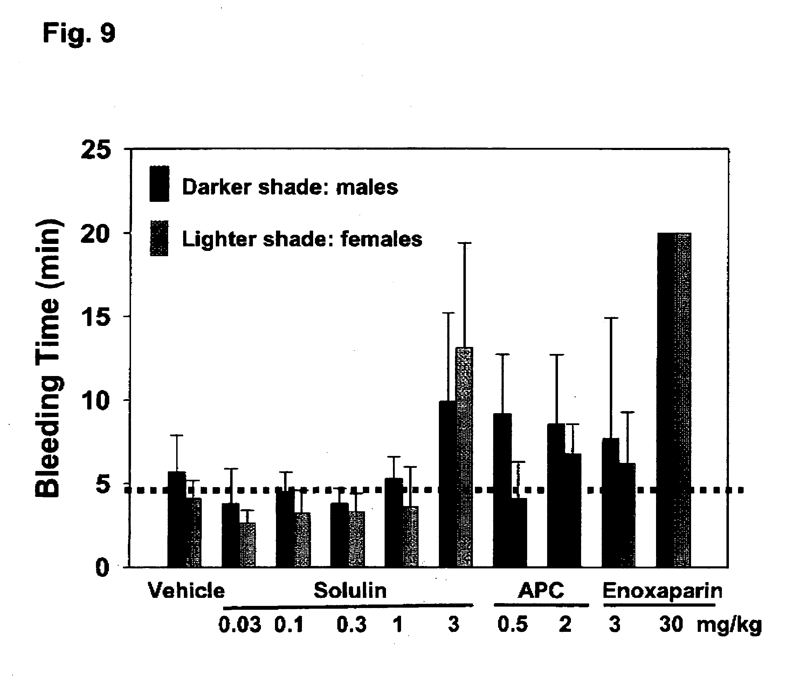

The results are summarized in tab. 2 and are shown in fig. 9 (Bleeding Time in Rats). Solulin significantly prolonged bleeding time in both sexes only at the highest dose of 3 mg/kg. This effect was more pronounced in females than in males. Incidences of re-bleedings in Solulin-treated males were similar to controls ranging between 20 and 40 %. In females of the Solulin groups, the rate of re-bleedings was slightly elevated (up to 60 %), but were lacking any dose-dependency.

APC significantly extended the bleeding time at 0.5 mg/kg in males and at 2 mg/kg in both genders. After administration of the 0.5 mg/kg dose, re-bleadings remained comparable to controls (20-40 %), but increased to 60 % in males and 80 % in females in the 2 mg/kg group.

Enoxaparin significantly prolonged bleeding time, at 3 mg/kg in females, and maximally, i.e. until the end of the observation period, at 30 mg/kg in males and females. In males of the 3 mg/kg Enoxaparin group, the incidence of re-bleedings was equivalent to controls. However, re-bleedings were evident in 80 % of the females receiving the same dose. As the bleeding time was maximally prolonged by 30 mg/kg Enoxaparin in both sexes, the rate of re-bleedings could not be assessed.

**Table 2: Effects of various Solulin doses compared to APC and Enoxaparin on tail transection bleeding time in rats.**

| **Substance** | | **Bleeding Time** | | | | | |
|---|---|---|---|---|---|---|---|
| | **Dose [mg/kg]** | **Males** | | | **Females** | | |
| | | **[min]** | **% prolongation** | **% rebleeding** | **[min]** | **% prolongation** | **% rebleeding** |
| Vehicle | 5 ml/kg | 5.7 ± 2.2 | - | 40 | 4.1 ±1.1 | - | 20 |
| Solulin | 0.03 | 3.8 ± 2.1 | 0 | 20 | 2.6 ± 0.8 | 0 | 20 |
| | 0.1 | 4.5 ± 1.2 | 0 | 20 | 3.2 ± 1.4 | 0 | 60 |
| | 0.3 | 3.8 ± 0.9 | 0 | 40 | 3.3 ± 1.1 | 0 | 40 |
| | 1 | 5.3 ± 1.3 | 0 | 20 | 3.6 ± 2.4 | 0 | 60 |
| | 3 | 9.9 ± 5.3* | 75 | 40 | 13.1 ± 6.3* | 223 | 40 |
| APC | 0.5 | 9.2 ± 3.5* | 63 | 40 | 4.1 ± 2.2 | 2 | 20 |
| | 2 | 8.6 ± 4.1* | 52 | 60 | 6.8 ± 1.8* | 67 | 80 |
| Enoxaparin | 3 | 7.6 ± 7.2 | 34 | 40 | 6.2 ± 3.1* | 53 | 80 |
| | 30 | 20.0 ± 0* | 253 | - | 20.0 ± 0* | 393 | - |

| | | | | | | | |
|---|---|---|---|---|---|---|---|
| * p < 0.05 by one-way ANOVA followed by Dunnett's test: % reduction compares the value to the respective vehicle group; data represent mean values ± SEM of 5 males and 5 females each. | | | | | | | |

From these results and the pharmacodynamic studies reported above, it can be concluded that Solulin poses no sizable risk of bleeding, in particular at dosages that are efficacious in rats and mice.

That APC significantly affected the bleeding time even at doses that were either barely (0.5 mg/kg) or moderately effective (2 mg/kg) in the rat stasis model of venous thrombosis is in agreement with the clinical profile of this drug and may be explained by a more general inhibition of thrombin generation.

While the bleeding rate was acceptable following administration of the Enoxaparin low dose, this study also confirms that Enoxaparin, at adose of 30 mg/kg, substantially affects coagulation and hence seems to be at the upper limit of tolerability. It should be noted, that one female of the APC and Enoxaparin high dose groups each died within 24 h of the experiment while no fatalities were observed with Solulin treatment.

### V. EXAMPLE

### Effects on coagulation parameters in vivo

In order to obtain a more complete picture of the pharmacodynamics of Solulin, APC (Xigris^{®} (Drotrecogin alfa activated) and Enoxaparin at the doses used in the venous stasis model, the effects of these drugs on coagulation parameters were evaluated *in vivo.*

### 1. Test System and Experimental Procedures

Solulin, Enoxaparin or vehicle (0.9% NaCl) were i.v, administered to male and female rats 30 min before blood collection from the inferior vena cava. APC was i.v. applied 5 min before blood collection from the same site. Citric platelet-poor plasma was prepared and tested for activated Partial Thromboplastin Time (aPTT), Prothrombin Time (PT) and Thrombin Time (TT).

### 2. Results

All three drugs significantly affected PT at the highest dose leading to a mild 1.1-fold prolongation except Enoxaparin-treated females which showed no effect (tab. 3).

**Table 3: Effects of various Solulin doses compared to APC and Enoxaparin on coagulation in vivo.**

| **Substance** | **Dose [mg/ kg]** | **Males** | | | **Females** | | |
|---|---|---|---|---|---|---|---|
| | | **PT [sec]** | **TT [sec]** | **aPTT [sec]** | **PT [sec]** | **TT [sec]** | **aPTT [sec]** |
| Vehicle | 5 ml/kg | 10.9 ± 0.17 | 47.4 ± 3.11 | 16.1 ± 0.39 | 10.4 ± 0.16 | 41,6 ± 0.43 | 16.8 ± 1.09 |
| Solulin | 0.03 | 10.7 ± 0.08 | 44.7 ± 1.33 | 15.3 ± 0.25 | 10.2 ± 0.09 | 44.2 ± 0.31 | 16.4 ± 0.73 |
| | 0.1 | 10,9 ± 0.2 | 50.0 ± 1.41 | 15.7 ± 0.32 | 10.2 ± 0.22 | 47.8 ± 0.55 | 17.4 ± 0.76 |
| | 0.3 | 11.1 ± 0.23 | 71.8 ± 6.48* | 17.8 ± 1.05 | 10.6 ± 0.24 | 80.1 ± 8.06* | 18.8 ± 0.47 |
| | 1 | 11.4 ± 0.29 | 184.4 ± 15.91* | 20.7 ± 1.11 | 10.7 ± 0.11 | 188.6 ± 15.52* | 23.3 ± 0.43* |
| | 3 | 11.9 ± 0.34* | 600.0 ± 0* | 25.2 ± 1.06* | 11.4 ± 0.21* | 600.0 ± 0* | 29.3 ± 0.79* |
| APC | 0.5 | 11.5 ± 0.17 | 45.8 ± 0.74 | 16.8 ± 0.52 | 10.8 ± 0.1 | 45.8 ± 1.98 | 19.1 ± 1.41 |
| | 2 | 12.2 ± 0.07* | 41.5 ± 0.91 | 25.9 ± 2.92* | 11.4 ± 0.11* | 46.9 ± 2.49 | 26.9 ± 1.19* |
| Enoxaparin | 3 | 11.1 ± 0.18 | 289.6 ± 5.48* | 29.2 ± 1.05* | 10.7 ± 0.19 | 340.9 ± 17.78* | 31.1 ± 2.20* |
| | 30 | 12.0 ± 0.18* | 600.0 ± 0* | 600.0 ± 0* | 10.7 ± 0.09 | 600.0 ± 0* | 600.0 ± 0* |

| | | | | | | | |
|---|---|---|---|---|---|---|---|
| * p < 0.05 by one-way ANOVA followed by Dunnetfs test; data represent mean values ± SEM of 5 males and 5 females each. | | | | | | | |

Solulin and Enoxaparin dose-dependently influenced TT, leading to a maximal prologation at the respective high doses. Effects were significant at 0.3-3 mg/kg Solulin and at both Enoxaparin doses corresponding to 1.5 -14.4-fold and 6.1-14.4-fold prolongations, respectively. in contrast. APC did not prolong TT in males and barely affected it in females (tab. 3).

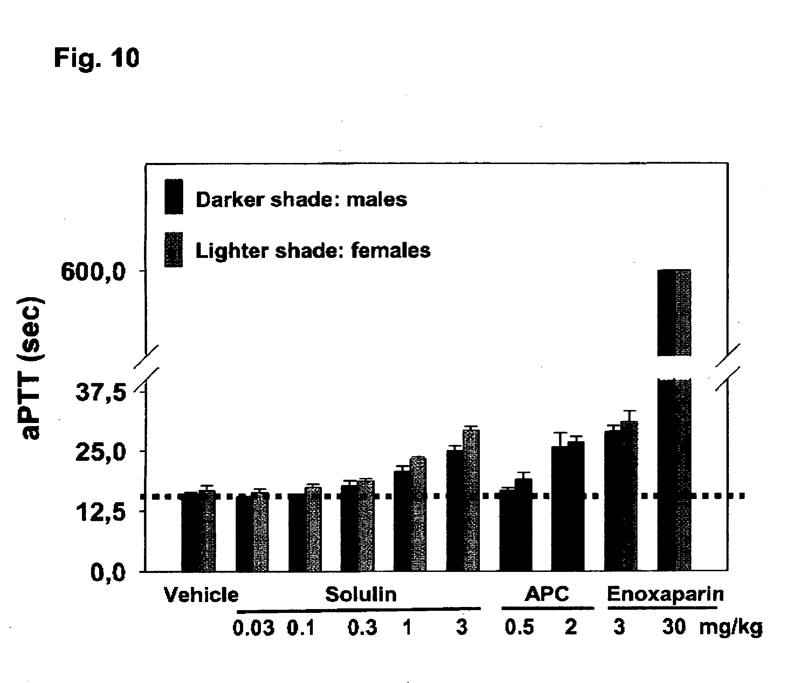

Solulin significantly prolonged aPTT at 1 mg/kg in females and in both genders treated with the 3 mg/kg dose, causing a 1.4-1.7-fold aPTT prolongation. APC (2 mg/kg) significantly increased aPTT to a comparable extent (1.6-fold). Also both doses of Enoxaparin significantly influenced aPTT. However, while the increase by the 3 mg/kg dose fell in the same range as that seen with the other two drugs (1.8-fold), 30 mg/kg Enoxaparin caused an excessive prolongation of aPTT (more than 37-fold) compared to vehicle treatment (tab. 3, fig. 10 (Prolongation of aPTT in Rats)).

It should be noted that a 1.5- to 2.5-fold aPTT prolongation was formerly used as the efficacy level to which unfractionated heparin was titrated. More recently, even 2.0- to 3.5-fold prolongations have been recommended in case the heparin dose cannot be calibrated by means of protamine titration or the anti-factor Xa assay, because variability among aPTT reagents and coagulometers used in different laboratories had resulted in insufficient heparin exposure. Although Enoxaparin is a low molecular variant of heparin, the drastic effect of the 30 mg/kg dose on aPTT would most probably be intolerable which is substantiated by the high risk determined in the bleeding time study. Nevertheless, the effects of all Solulin or APC dosages meet the acceptable aPTT range.

## Claims

1. Use of thrombomodulin for the manufacture of a medicament for stimulating the reperfusion of an ischemic tissue or organ within a patient in the need thereof, in particular a stroke patient.

2. Use of thrombomodulin for the manufacture of a medicament for preventing an occlusion of blood vessels by thromboembolic events.

3. Use according to claim 2, whereas the occlusion is caused by a secondary thromboembolic event within the patient, in particular within a stroke patient.

4. Use according to claim 2 or 3, whereas the patient is treated within 48 hours after the onset of the thromboembolic event, preferably within 24 hours.

5. Use according to claim 4 whereas the treatment is within the first three hours after the onset of the thromboembolic event.

6. Use according to one of the above claims, whereas the thrombomodulin is administered in a dosage, which effectively prevents vessel occlusion and does not substantially increase blood coagulation as measured by the APTT assay.

7. Use according to claim 6, whereas thrombomodulin as administered in a dosage of 0.01 to 5 mg/kg body weight, preferably 0.03 to 3 mg/kg body weight, most preferred 0.03 to 1.6 mg/kg body weight or 0.05 to 1.4 mg/kg body weight.

8. Use according to any of the above claims, whereas the thrombomodulin has an amino acid sequence corresponding to the amino acid sequence of mature soluble thrombomodulin and comprises one or more of the subsequent modifications:
removal of amino acids 1-3
M388L
R456G
H457Q
S474A, and terminating at P490.
